# EUROPEAN PATENT APPLICATION

(11) **EP 3 299 032 A1**
(43) Date of publication of application: **28.03.2018**
(21) Application number: 16190362.0
(22) Date of filing: 23.09.2016
(51) Int. Cl.: A61K 45/00, A61P 29/00

(54) **INHIBITORS OF GPR132 FOR USE IN PREVENTING AND/OR TREATING CHEMOTHERAPY-INDUCED NEUROPATHIC PAIN**

(71) Applicant: Fraunhofer-Gesellschaft zur Förderung der angewandten Forschung e.V., 80686 München (DE); Goethe-Universität Frankfurt am Main, 60323 Frankfurt am Main (DE)
(72) Inventor: Schiffmann, Susanne., 63263 Neu-Isenburg (DE)
(74) Representative: Herzog, Fiesser & Partner Patentanwälte PartG mbB

(57) **Abstract**

The present invention relates to an inhibitor of GPR132 for use in preventing and/or treating chemotherapy-induced neuropathic pain in a subject. Also encompassed by the invention are a pharmaceutical composition comprising an inhibitor of GPR132 as well as a method for identifying an inhibitor of GPR132.

## Description

The present invention relates to an inhibitor of GPR132 for use in preventing and/or treating chemotherapy-induced neuropathic pain in a subject. Also encompassed by the invention are a pharmaceutical composition comprising an inhibitor of GPR132 as well as a method for identifying an inhibitor of GPR132.

Chemotherapy-induced neuropathic pain also referred to as chemotherapy-induced peripheral neuropathy (CIPN) is a severe adverse event of cytostatic drugs during cancer therapy. CIPN is one of the major reasons for delay or discontinuation of chemotherapy and therefore responsible for decreased chemotherapeutic efficacy and loss of quality of life (Sisignano et al., 2014) (Wang et al., 2012). Several compounds have been suggested to be useful in the treatment of CIPN (e.g. WO 2005/105135 A1, WO 2016/016178 A1). However, there are currently no approved or recommended agents for the prevention or effective treatment of CIPN (Hershman et al., 2014).

Platinum-based compounds such as cisplatin or oxaliplatin, vinca-alkaloids such as vincristine and vinblastine, taxanes such as paclitaxel and docetaxel as well as proteasome inhibitors such as bortezomib, are antineoplastic drugs which can induce a severe CIPN. Oxaliplatin is widely used as first-line treatment for colorectal carcinomas and induces CIPN in up to 80 % of patients (Pachman et al., 2011). Symptoms manifest in the extremities of the body and may proceed along the limbs during repetitive treatment (Pachman et al., 2011). Although CIPN might involve inflammatory processes, the key enzymes and signalling molecules seem to differ compared to classical inflammatory pain models. Treatment with oxaliplatin does not induce an increased expression of COX2 as well as no increase in prostaglandin synthesis, thus pointing towards a different mechanism of oxidized lipid synthesis and signalling (Sisignano et al., 2014). For this reason, non-steroidal anti-inflammatory drugs (NSAIDs) are not effective in treating oxaliplatin-induced CIPN (Park et al., 2013).

The molecular mechanisms behind the oxaliplatin-induced peripheral neuropathy are still unclear. It is suggested that ligand-gated ion channels like transient receptor potential (TRP)-channels may contribute to the pathology of CIPN. Under physiological conditions TRP-channels serve for the perception of external stimuli like heat or capsaicin from chilli pepper (TRPV1), low pH or allyl isothiocyanate from wasabi (TRPA1), cool temperatures or menthol from peppermint (TRPM8) as well as osmotic and mechanical stimulations (TRPA1, TRPV4, TRPC3 and TRPC6) (Moran et al., 2011). Under pathophysiological conditions TRP channels are sensitized, which causes a reduction of their activation threshold and thereby increased sensitivity to painful (hyperalgesia) or normally non-painful stimuli (allodynia) (Julius, 2013). These sensitization effects on TRP channels can be mediated by lipid mediators such as Prostaglandin E2 (PGE2), other eicosanoids or oxidized linoleic acid metabolites. These lipids bind to G-protein coupled receptors (GPCRs) in sensory neurons causing activation of various G-protein-dependent signaling pathways and leading to protein kinase A (PKA)- and PKCε-mediated phosphorylation of TRP-channels (Wang et al., 2008; Wang et al., 2015) causing their activation (Moriyama et al., 2005; Sisignano et al., 2014). Increased sensitization of TRPV1, TRPA 1 and TRPV4 may contribute to mechanical hypersensitivity and cold allodynia in chemotherapy-induced peripheral neuropathy (Nassini et al., 2011; Li et al, 2015).

G protein coupled receptor 132 (GPR132), also known as G-protein coupled receptor G2A (G2A receptor), is a member of the proton sensing G protein coupled receptor (GPR) subfamily. GPR132 was first identified as a stress-inducible GPCR (Weng et al., 1998). GPR132 is a seven transmembrane G protein coupled receptor and resides in the cell surface membrane. Lysophosphatidylcholine and sphingosylphosphorylcholine were initially reported to be direct ligands for GPR132. However, these studies did not give evidence that these phospholipids actually bound to GPR132 and consequently the paper was retracted four years later. Moreover, certain linoleic acid and arachidonic acid metabolites have been suggested to act as ligands for GPR132 (Obinata et al. 2005).

GPR132 expression is mainly found in blood leukocytes such as macrophages, dendritic cells, neutrophils, T lymphocytes and B lymphocytes and can be upregulated by stress and prolonged mitogenic signals. More recently, GPR132 was also described to be expressed in certain neuronal populations such as the dorsal root ganglion (DRG). Knockout mice lacking GPR132 (G2A) were reported to develop autoimmune diseases. Furthermore, G2A-deficient T cells were shown to be hyper-responsive to TCR stimulation, exhibiting enhanced proliferation and a lower threshold for activation.

Since GPR132 has been implicated in the regulation of the immune system, the focus on GPR132 as a therapeutic target, mainly in the sense of activating GPR132 signalling, has been on inflammatory diseases. WO2003/080071 A1 discloses agonist ligands specific to G2A receptor for treating a disease or disorder with neutrophil accumulation, excess release of IL-8 or with microbial infection. WO2008/156833 A2 discloses the G2A receptor as a therapeutic target for treating and preventing disease states characterized by decreased high-density lipoprotein (HDL) particle levels, increased very-low density lipoprotein (VLDL) particle or increased low-density lipoprotein (LDL) particle levels such as chronic inflammatory autoimmune disorders, atherosclerosis, hyperlipidemia or hypercholesterolemia. WO2004/003567 A1 relates to nucleic acid sequences and amino acid sequences of human G2A and its regulation for the treatment of hematological diseases, cardiovascular diseases, disorders of the peripheral and central nervous system, gastro-intestinal diseases and inflammation in mammals.

The use of an inhibitor of GPR132 for preventing and/or treating chemotherapy-induced neuropathic pain, and especially for neuropathic pain induced by a platinum-containing chemotherapeutic agent such as oxaliplatin, has not been disclosed nor is anticipated by the prior art.

Although CIPN might involve inflammatory processes, the key enzymes and signaling molecules seem to differ compared to classical inflammatory pain models. Treatment with oxaliplatin does not induce an increased expression of COX2 as well as no increase in prostaglandin synthesis, thus pointing towards a different mechanism of oxidized lipid synthesis and signaling (Sisignano et al., 2014). For this reason, non-steroidal anti-inflammatory drugs (NSAIDs) are not effective in treating oxaliplatin-induced CIPN.

As mentioned before, there are currently no therapies with a confirmed neuroprotective benefit available for clinical use in patients with chemotherapy-induced neuropathic pain (CIPN) and especially for patients with neuropathic pain induced by the widely used platinum-containing chemotherapeutic agents such as oxaliplatin.

In view of the above, an ongoing demand exists for the development of therapeutic agents for use in the prevention and/or treatment of chemotherapy-induced neuropathic pain.

The technical problem underlying the present invention can be seen as the provision of means and methods for complying with the aforementioned needs. The technical problem is solved by the embodiments characterized in the claims and herein below.

The present invention pertains to an inhibitor of GPR132 (G protein coupled receptor 132) for use in preventing and/or treating chemotherapy-induced neuropathic pain in a subject, wherein said subject has received at least one platinum-containing chemotherapeutic agent.

The term "chemotherapy-induced neuropathic pain", also referred to as chemotherapy-induced peripheral neuropathy (CIPN) relates to neuropathic pain that is induced by a chemotherapeutic agent (also known as cytotoxic or cytostatic agents). CIPN is a severe debilitating side effect of widely used cytostatics during cancer therapy, for example platinum-based compounds such as cisplatin or oxaliplatin, vinca-alkaloids such as vincristine and vinblastine, taxanes such as paclitaxel and docetaxel and proteasome inhibitors such as bortezomib. Typical symptoms of CIPN include pain, tingling, numbness and temperature sensitivity. Sometimes, also motor nerves/central nervous system and/or the autonomic nervous system are affected. Typically, the nerve endings in the extremities of the hands and feet are affected earliest by toxicity in a symmetrical, length-dependent manner. Large sensory nerve fibers are most commonly affected, with damage to smaller sensory fibers occurring only rarely. Sensory nerve dysfunction with symptoms such as sensory ataxia, pain, and severe numbness is generally more common than motor involvement. However, motor and autonomic neuropathic symptoms may also develop. Currently, there are no therapies with a confirmed neuroprotective benefit available for clinical use in patients with CIPN. To date, CIPN is typically alleviated by dose reduction of the used chemotherapeutic agent(s), which may compromise treatment efficacy. Further symptoms and characteristics of CIPN are well known in the art and are, for example, described in the standard text books of medicine, such as Stedman or Pschyrembl.

Preferably, the chemotherapy-induced neuropathic pain is associated with the administration of a platinum-containing chemotherapeutic agent, more preferably, with the administration of oxaliplatin. The term "associated with" as used herein, preferably, refers to a temporal and/or causal relationship between neuropathic pain and the administration of a platinum-containing chemotherapeutic agent. The person skilled in the art understands what is meant if the pain is considered to be associated with the administration of a platinum-containing chemotherapeutic agent. Preferably, the pain shall be considered to be associated with the administration of a platinum-containing chemotherapeutic agent if the pain is caused, i.e. induced by said the administration of a platinum-containing chemotherapeutic agent, whether directly or indirectly. Indication for such causal connection is in particular a close time-relationship between the administration and the pain.

The term "platinum-containing chemotherapeutic agent" as used herein refers to a chemotherapeutic agent to treat cancer that comprise platinum, preferably one or more coordination complexes of platinum. Such drugs are commonly also referred to as "platins" or "platinum-based antineoplastic drugs". Platinum-containing chemotherapeutic agents are well known in the art. Preferably, the agent is selected from the group consisting of cisplatin, carboplatin, oxalipaltin, nedaplatin, triplatin tetranitrate, phenanthriplatin, picoplatin, satraplatin and variants thereof such as Lipoplatin (Liposomal cisplatin). The administration and dosage of platinum-containing chemotherapeutic agents depends on various factors such as the kind and state of the cancer to be treated and the health state of the patient. Platinum-containing chemotherapeutic agents are usually administered under the supervision of a qualified physician experienced in the use of platinum-containing chemotherapeutic agents. Preferred administration routes of platinum-containing chemotherapeutic agents include intravenous, intrathecal and intraperitoneal administration. Several, potential dose-limiting, side effects of platinum compounds are known in the art including acute neurotoxicity and CIPN.

Preferably, the platinum-containing chemotherapeutic agent is oxaliplatin. Oxaliplatin is a third-generation platinum analog that features a square planar platinum(II) center. In contrast to cisplatin and carboplatin, oxaliplatin features the bidentate ligand 1,2-diaminocyclohexane in place of the two monodentate ammine ligands and also features a bidentate oxalate group. Oxaliplatin has been shown to be active against both early-stage and advanced colorectal cancer. For treatment of colorectal cancer, oxaliplatin is often used together with folinic acid and 5-fluorouracil in a combination (known as FOLFOX).

The term "treating" as used herein refers to ameliorating or curing a disease or at least one symptom associated therewith. Preferably said disease is CIPN or at least one symptom associated therewith. Thus, if there is amelioration or cure of the disease or at least a symptom associated therewith, the treatment shall be deemed to be effective. It will be understood that treating might not be effective in all subjects. However, according to the present invention it is envisaged that treatment will be effective in at least a statistically significant portion of subjects to be treated. It is well known to the skilled artisan how to determine a statistically significant portion of subjects that can be effectively treated.

Whether a portion of subjects or any other value referred to herein is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98% or at least 99%. The p-values are, preferably, 0.1, 0.05, 0.01, 0.005, or 0.0001.

Preferably, the probability envisaged by the present invention allows that the finding of effective treatment will be correct for at least 60%, at least 70%, at least 80%, or at least 90% of the subjects of a given cohort or population.

The term "preventing" as used herein refers to avoiding the onset of a disease or at least one symptom thereof. Preferably said disease is CIPN. It will be understood by those skilled in the art, that CIPN as used herein also includes pre-CIPN states with no or very weak symptoms in which one or more of the symptoms required to label a person as having CIPN are present and where peripheral nerve damage has not yet occurred, but the risk of developing CIPN in a subject is present. Preventive administration of an inhibitor of GPR132 for use according to the present invention is especially useful in patients having an increased risk to suffer from CIPN, for example diabetic patients, patients with hereditary neuropathies or patients who already show neuropathic symptoms due to acquired immune-deficiency syndrome (AIDS). Preferably, for preventing CIPN, an inhibitor of GPR132 is given after the subject has received the first dose of at least one platinum-containing chemotherapeutic agent and before one or more symptoms of CIPN are present.

GPR132 (G protein coupled receptor 132), also known as G-protein coupled receptor G2A (G2A receptor), is a seven transmembrane G-protein coupled receptor that resides in the cell surface membrane. GPR132 was first identified as a stress-inducible G-protein coupled receptor (Weng et al., 1998). The nucleic acid and protein sequences of GPR132 can be found in public databases, for example protein sequences of human and mouse GPR132 can be found in the UniProtKB database under the Accession Numbers Q9UNW8 (GP132_HUMAN) and Q9Z282 (GP132_MOUSE), respectively.

The term "inhibitor of GPR132" refers to any substance that is capable of reducing, partially impairing and/or fully inhibiting the activity of GPR132. It will be understood that GPR132 activity refers to the capability of GPR132 to activate downstream signaling molecules, for example G proteins, protein kinases and phospholipases. Typically, a G protein-coupled receptor such as GPR132 is activated by an external signal in the form of a ligand or other signal mediator. This creates a conformational change in the receptor, causing activation of a G protein which further mediate G-protein dependent signaling such as activation of protein kinases, for example the activation of protein kinase C (PKC) which further leads to PKC-mediated phosphorylation of TRP-channels and/or the release of calcium from the endoplasmic reticulum. Thus, inhibiting the activity of GPR132 can also mean inhibiting processes downstream of GPR132, in particular, the release of calcium from the endoplasmic reticulum. Inhibition of GPR132 activity can be achieved by direct or indirect impairment of GPR132 activity. For example, direct inhibition of GPR132 can be achieved by binding of an antagonistic small molecule or antibody to the GPR132 protein located in the cell membrane. Such a direct inhibitor can be reversible or non-reversible bound to GPR132. Indirect inhibition can be achieved by inhibition of a GPR132-activating molecule, for example by inhibition of oxidized lipids such as 9-HODE that is known to activate GPR132. It will be understood that an inhibitor of GPR132 can also be an allosteric inhibitor, for example, a molecule that binds to a different site at the receptor than an activator and therefore acts as allosteric inhibitor. It will be further understood that inhibition of GPR132 activity can take place at the DNA, RNA or protein level. Preferably, said inhibition is achieved be decreasing the expression of the DNA, RNA and/or protein. For example, inhibitors of GPR132 can be molecules that lead to tight packaging of DNA or are transcriptional inhibitors, respectively, so that GPR132 mRNA cannot be made. Included as inhibitors of GPR132 are for example also molecules that interfere with the transcriptional upregulation of GPR132 that is often observed after administration of certain chemotherapeutic agents. Inhibitors of GPR132 can also be post-transcriptional inhibitors, meaning that the GPR132 protein is not correctly translated, transported or inserted into the cell membrane and thus, GPR132 does not function properly. Preferably, said inhibitor of GPR132 for use according to the present invention is a direct inhibitor of GPR132. More preferably, said inhibitor of GPR132 is a peptide or protein, a ribozyme, an antibody, a small molecule, an inhibitory RNA molecule, an antisense oligonucleotide, a morpholino, or a lipid, in particular lysophosphatidylcholine (LPC).

A peptide or protein as referred to herein relates to a molecule consisting of amino-acid residues joined by peptide bonds. Peptides, consisting of several, typically, at least 20, at least 30, at least 40, at least 50 or at least 60 amino acids that are covalently linked to each other by peptide bonds, are commonly referred to as polypeptides. Molecules consisting less than 20 amino acids covalently linked by peptide bonds are usually considered to be peptides. In a preferred embodiment, the peptide or protein shall be a direct or indirect inhibitor of GPR132. More preferably, the peptide or protein shall directly bind to GPR132, thereby inhibiting its activity.

A ribozyme as referred to herein relates to an RNA molecule that is capable of catalyzing specific biochemical reactions, including cleavage and/or ligation of RNA and DNA and peptide bond formation. Methods of designing and constructing ribozymes are known in the art and include, for example, de novo rational design, oligonucleotide synthesis and in vitro-transcription. It is also known in the art that ribozymes can be stably integrated or transiently introduced into cells as part of a recombinant DNA construct such as a plasmid or vector. It will be understood that such a DNA construct may contain additional regulatory elements such as an enhancer, a constitutive or inducible promoter or a terminator. The ribozyme shall be a direct or indirect inhibitor of GPR132. In a preferred embodiment, the ribozyme specifically targets the GPR132 mRNA which is transcribed from the gene encoding GPR132, followed by endonucleolytic cleavage of GPR132 mRNA. Consequently, no functional GPR132 protein can be made from said cleaved mRNA and thus GPR132 activity is inhibited.

An antibody as referred to herein include both polyclonal and monoclonal antibodies, as well as fragments thereof, such as Fv, Fab and F(ab)2 fragments that are capable of binding an antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. The antibody shall inhibit the activity of GPR132, for example by direct binding to GPR132 itself or by indirect interaction with GPR132, for example by binding to a GPR132 activator such as 9-HODE and thereby inhibiting the activation of GPR132. In a preferred embodiment, the antibody is an antagonistic antibody specifically binding to GPR132 and thereby inhibiting GPR132 activity. Preferably the antibody specifically binds to an epitope comprised by extracellular domain of GPR132, thereby inhibiting GPR132 activity. More preferably, the antibody may bind to an epitope comprised by the by the first 45 amino acids of human GPR132 (UniprotKB Accession No. Q9UNW8) or comprised by the first 42 amino acids of mouse GPR132 (UniprotKB Accession No. Q9Z282). Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the binding agent can be measured by any method known in the art. Preferably, said method is semi-quantitative or quantitative.

A small molecule as referred to herein relates to a molecule with a low molecular weight. Typically, small molecule is an organic compound with a molecular weight of less than 900 daltons. Small molecules include, for example, small secondary metabolites such as alkaloids, lipids, gylcosides, terpenes, tetrapyrroles, phenazines, oliogonucleotides and small peptides or peptide-like molecules. The small molecule shall be a direct or indirect inhibitor of GPR132. In a preferred embodiment, the small molecule directly binds to GPR132, thereby inhibiting its activity.

An inhibitory RNA molecule as referred to herein is an RNA molecule that inhibits gene expression in a sequence-specific manner. Inhibitory RNA molecules include, for example, small interfering RNA (siRNA), small hairpin RNAs (shRNA) and microRNA (miRNA). The inhibitory RNA molecule typically induces a process known as RNA interference (RNAi), leading to cleavage and/or translational inhibition of a target mRNA with a complementary sequence. The inhibitory RNA molecule can show perfect or imperfect base-pairing to a complementary target sequence. siRNA and shRNAs typically base-pair perfectly and induce mRNA cleavage only in a single, specific target. MiRNAs typically typically show incomplete base pairing to a target and often inhibit the translation of many different mRNAs with similar sequences. An inhibitory RNA molecule may also induce a process known as Means and methods to design and produce inhibitory RNA molecules are well known in the art. An inhibitory RNA molecule may be chemically synthesized or expressed within the cell, for example by introduction of respective recombinant DNA construct. It will be understood that such a DNA construct may contain additional regulatory elements such as an enhancer, a constitutive or inducible promoter or a terminator. The inhibitory RNA molecule shall be a direct or indirect inhibitor of GPR132. In a preferred embodiment, the inhibitory RNA molecule directly interferes with the GPR132 mRNA. Thereby translation of GPR132 mRNA into a functional protein is prevented and inhibition of GPR132 signaling is achieved. In yet a preferred embodiment, the inhibitory RNA molecule interferes with the mRNA of an activator and/or ligand of GPR132, thereby preventing activation of GPR132 by the activator and/or ligand.

An antisense oligonucleotide as referred to herein relates to a single strand DNA and/or RNA molecule that is capable of interfering with DNA and/or RNA processing. Antisense oligonucleotides comprise a nucleic acid sequence which is complementary to a specific RNA or DNA sequence. Typically, an antisense oligonucleotide will bind, in a sequence-specific manner, to their respective complementary oligonucleotides, DNA, or RNA, thereby interfering with DNA and/or RNA processing. Antisense oligonucleotides may interfere with mRNA processing through RNase H-mediated degradation, translational arrest, modulation of splicing or they may act through steric hindrance of proteins, e.g. sterical hindrance of Serine-Arginine-rich proteins to exonic splicing enhancer sites. Means and methods for the design and synthesis of antisense oligonucleotides are well known in the art and include, for example, rational design, design of antisense oligonucleotides containing locked nucleic acids (LNA) and solid-phase chemical synthesis. Antisense oligonucleotides can be chemically synthesized or expressed within the cell, for example by introduction of respective recombinant DNA construct. It will be understood that such a DNA construct may contain additional regulatory elements such as an enhancer, a constitutive or inducible promoter or a terminator. Preferably, the antisense oligonucleotide has a length of at least 8, at least 10, at least 12, at least 15, at least 20, at least 25, at least 30, at least 40, at least 45, at least 50 or more nucleotides. The antisense oligonucleotide may comprise deoxyribonucleotides, ribonucleotides, or a combination of both. The antisense oligonucleotide shall be a direct or indirect inhibitor of GPR132. In a preferred embodiment, the antisense oligonucleotide is a DNA and/or RNA molecule that interferes with expression and/or translation of GPR132. In yet a preferred embodiment, the antisense oligonucleotide is a DNA and/or RNA molecule that interferes with expression and/or translation of an activator and/or ligand of GPR132, thereby preventing activation of GPR132 by the activator and/or ligand.

A morpholino as referred to herein relates to a molecule that blocks access of other molecules to small, specific sequences, typically around 25 bases, of the base-pairing surfaces of an RNA. A morpholino usually comprises a backbone of methylenemorpholine rings and phosphorodiamidate linkages. Morpholinos are commonly also known as morpholino oligomers (MO nucleic acid analogs) and phosphorodiamidate morpholino oligomers (PMO). In contrast to many other antisense structural molecules such as phosphorothioates or siRNA, Morpholinos do not degrade their target RNA molecules. Instead, Morpholinos act by sterical blocking, i.e. binding to a target sequence within a RNA and simply getting in the way of molecules that may otherwise interact with the RNA. The morpholino shall be a direct or indirect inhibitor of GPR132. In a preferred embodiment, the morpholio is binding to the GPR132 pre-mRNA and/or mRNA. By binding to the pre-mRNA and/or mRNA, the morpholino can interfere with GPR132 splicing and/or translation causing production of a less or non-functional GPR132 protein, a reduced GPR132 protein production or no GPR132 protein production at all, thereby inhibiting GPR132 activity.

A lipid as referred to herein relates to hydrophobic or amphiphilic small molecules. Lipids include fatty acids and their derivates such as triglycerides, diglycerides, monoglycerides, phospholipids, lysophospholipids such as lysophosphatidylcholin (LPC), glycerolipids, glycerophospholipids, sphingolipids, saccharolipids, polyketides, prenol lipids and sterol lipids. The lipid shall be a direct or indirect inhibitor of GPR132. In a preferred embodiment, the lipid directly interacts with the GPR132 protein, thereby inhibiting its activity. In yet a preferred embodiment the lipid interacts with an activator and/or ligand of GPR132 such as 9-HODE, thereby inhibiting the binding of the activator and/or ligand to GPR132, or it binds to a different site at the receptor than an activator, such as 9-HODE, and act as allosteric inhibitor.

The term "lysophospholipid" refers to any derivative of a phospholipid in which one or both acyl derivatives have been removed by hydrolysis. Lysophospholipids include lysophosphatidylcholine, lysoglycerophospholipid, lysoglycosphingolipid, lysolipid, lysolecithin, lysophosphatidylethanolamine, lysocephalin, lysophosphatidylinositol, lysophosphatidylserine, and lysosphingomyelin. Preferably, the lysophospholipid is lysophosphatidylcholine (LPC). The term "lysophosphatidylcholine (LPC)" relates to a lysophospholipid that typically results from partial hydrolysis of a phosphatidylcholine. The hydrolysis is typically carried out by phospholipase A2 (PLA2), wherein the PLA2 removes one of the fatty acid groups of the phosphatidylcholine. In vivo, LPCs may be quickly metabolized by lysophospholipase and LPC-acyltransferase, thus usually having a short half live in vivo. It has recently been shown that the G2A receptor (GPR132) can be antagonized by certain lysophosphatidylcholine species (Murakami et al, 2004). It will be understood that the term lysophosphatidylcholines also includes LPC analogues, for example an LPC analogue where an acyl-group within the LPC is replaced with an alkyl-group, also referred to as alkyl-lysophospholipid (ALP), which leads to increased metabolic stability. LPC analogues are known in the art and include, for example, edelfosine, miltefosine and perifosine. Preferably, the LPC inhibits the activity of GPR132, i.e. functions as an antagonist of GPR132. More preferably, said LPC is LPC (18:1), LPC (18:0), LPC (18:2), LPC (16:0), or LPC (20:4).

Preferably, the inhibitor of GPR132 reduces chemotherapy-dependent hyper-excitability of sensory neurons. Sensory neurons are nerve cells that transmit sensory information such as feeling, sight or sound. Sensory neurons are activated by sensory input, and send projections to other elements of the nervous system, ultimately conveying sensory information to the brain or spinal cord. Hyper-excitability of sensory neurons means that the threshold for activating sensory neurons is lower than usual. Thus, a "normal" signal can lead to an excessive reaction to stimuli. Means and methods to determine the excitability of sensory neurons are known in the art and include for example measurements of action potential generation, synaptic transmission and opening of ion channels (i.e. sodium, calcium).

Hyper-excitability of sensory neurons is often observed in cancer patients that undergo chemotherapy. According to the present invention, chemotherapy-dependent hyper-excitability of sensory neurons is preferably observed in a subject that has received a platinum-containing chemotherapeutic agent, more preferably oxaliplatin.

Preferably, the inhibitor of GPR132 reduces chemotherapy-dependent sensitization of a transient receptor potential (TRP) channel. TRP channels are a group of ion channels mainly located on the plasma membrane. Most TRP channels are composed of six membrane-spanning helices with intracellular N- and C-termini. Mammalian TRP channels are activated and regulated by a wide variety of stimuli and are expressed throughout the body. TRP channels mediate a variety of sensations like the sensations of pain, hotness, warmth or coldness, different kinds of tastes, pressure, and vision. TRP channels can be divided into seven subfamilies: (1) TRPC (canonical), (2) TRPV (vanilloid), (3) TRPA (ankyrin), (4) TRPM (melastatin), (5) TRPP (polycystin), (6) TRPML (mucolipin), and (7) TRPN (NOMPC) (Islam MS, ed., January 2011). Transient Receptor Potential Channels. Advances in Experimental Medicine and Biology. 704. Berlin: Springer. p. 700).

Under physiological conditions TRP-channels serve for the perception of external stimuli like heat or capsaicin from chilli pepper (TRPV1), low pH or allyl isothiocyanate from wasabi (TRPA1), cool temperatures or menthol from peppermint (TRPM8) as well as osmotic and mechanical stimulations (TRPA1, TRPV4, TRPC3 and TRPC6) (Moran et al., 2011). Under pathophysiological conditions TRP channels are sensitized, meaning that their activation threshold is reduced. As a result, increased sensitivity to painful (hyperalgesia) or normally non-painful stimuli (allodynia) can occur (Julius, 2013). Sensitization of TRP-channels can occur after administration of a chemotherapeutic agent. Chemotherapy-dependent sensitization of a TRP channel therefore means, that sensitization of at least one TRP-channel has occurred after administration of at least one chemotherapeutic agent, preferably at least one platinum-containing chemotherapeutic agent, more preferably, oxaliplatin. Further preferred is that chemotherapy-dependent sensitization has occurred to a TRP channel selected from the group consisting of: TRPV1, TRPA1, TRPM8, TRPV4, TRPC3 and TRPC6.

Chemotherapy-dependent sensitization of a TRP channel can be mediated by lipid mediators such as Prostaglandin E2 (PGE2), other eicosanoids or oxidized linoleic acid metabolites. These lipid mediators bind to G-protein coupled receptors (GPCRs) in sensory neurons causing activation of various G-protein-dependent signaling pathways and leading to protein kinase A (PKA)- and PKCε-mediated phosphorylation of TRP-channels (Wang et al., 2008; Wang et al., 2015), causing their activation (Moriyama et al., 2005; Sisignano et al., 2014; Watanabe et al., 2003; Basora et al, 2003, Wen et al, 2012).

According to the present invention, chemotherapy-dependent sensitization of a TRP channel is preferably mediated by oxidized lipids. For example, chemotherapy-dependent sensitization of TRPV1 may preferably be mediated by 9-HODE, chemotherapy-dependent sensitization of TRPA1 may be mediated Prostaglandin A2 (PGA2), 15-Deoxy-Delta-12,14-prostaglandin J2 (15-d-PGJ2), 5,6-epoxyeicosatrienoic acid (5,6-EET) or 4-hydroxynonenal (4-HNE), chemotherapy-dependent sensitization of TRPV4 may be mediated by 8,9-epoxyeicosatrienoic acid (8,9-EET) and chemotherapy-dependent sensitization of TRPC6 may be mediated by 20-hydroxyeicosatetraenoic acid (20-HETE). More preferably, said oxidized lipids are selected from the group consisting of: hydroxyoctadecadienoic acids (HODEs), dihydroxyoctadecenoic acids (DiHOMEs) and epoxyoctadecenoic acids (EpOMEs). Even more preferred, said oxidized lipids are selected from the group consisting of: 9-HODE, 13-HODE, 9,10-DiHOME, 12,13-DiHOME, 9,10-EpOME and 12,13-EpOME.

The term "subject" as used herein relates to animals, preferably to mammals such as mice, rats, sheep, dogs, cats, horses, monkeys, or cows and, more preferably, to humans. The terms "subject" and "patient" are used interchangeably herein.

A subject as used herein refers to a subject who has received at least one platinum-containing chemotherapeutic agent and is suspected to suffer from CIPN or to have an increases risk to develop CIPN and thus is in need for a therapy for preventing and/or treating CIPN. Preferably, the subject shows symptoms or clinical signs or parameters indicative for CIPN. The subject in accordance with the present invention shall have received at least one platinum-containing chemotherapeutic agent, preferably oxaliplatin, in the past, i.e. before administration of the inhibitor of GPR132. Preferably, the subject has received said inhibitor within 1 day, 2 days, 4 days, 1 week, 2 weeks, 3 weeks, 1 month, 2 months, 3 months, 4 months, 5 months or 6 months before administration of an inhibitor of GPR132.

Preferably, the subject is a patient in need of receiving at least one platinum-containing chemotherapeutic agent, preferably oxaliplatin. For example, it is envisaged that the subject suffers from colorectal cancer, in particular if the agent is oxaliplatin. It is understood that a patient suffering from colorectal cancer may have had other treatments before receiving at least one platinum-containing chemotherapeutic agent such as oxaliplatin and may show symptoms, clinical signs or other parameters related to colorectal cancer. Typical symptoms of colorectal cancer include changes in bowel habits, constipation, diarrhea, rectal bleeding or blood in stool, abdominal bloating, cramps or discomfort, stools that are thinner than normal, unexplained weight loss, nausea or vomiting, anemia, jaundice and weakness or fatigue. However, the term also relates to an apparently healthy subject who has received at least one platinum-containing chemotherapeutic agent, i.e. a subject not exhibiting any of the aforementioned symptoms, clinical signs or parameters. Especially patients diagnosed at an early stage of colorectal cancer often appear healthy and do not exhibit any of the aforementioned symptoms. At an early stage, colorectal cancer is often detected during routine healthcare or regular screening using colonoscopy. Preferably, said apparently healthy subject is a subject with an increases risk of developing CIPN, for example a diabetic patient or a patient with hereditary neuropathies.

Administration of therapeutic agents to said subject can be done in several ways including parental and/or oral administration. It is known to the skilled artisan, that administration and dosage of a therapeutic agent depends on various factors such as the health state of the subject and the disease to be treated. Preferably, said platinum-containing chemotherapeutic agent is administered intravenous, intrathecal and intraperitoneal administration, while said inhibitor of GPR132 for use according to the present invention is preferably given to said subject by oral and/or parental administration. Preferably, said platinum-containing chemotherapeutic agent to be administered to said subject is oxaliplatin. Said inhibitor of GPR132 to be administered to said subject preferably is a peptide or protein, a ribozyme, an antibody, a small molecule, an inhibitory RNA molecule, an antisense oligonucleotide, a morpholino, or a lipid, in particular lysophosphatidylcholin (LPC).

Therapeutic efficacy and toxicity of an inhibitor of GPR132 for use according to the present invention can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and by clinical factors. As is well known in the medical arts, dosages for any one patient depends upon many factors, including the patient's size, age, the particular formulation of the medicament to be administered, sex, time and route of administration, general health, and other drugs being administered concurrently. Progress can be monitored by periodic assessment. Dosage recommendations shall be indicated in the prescribers or users instructions in order to anticipate dose adjustments depending on the considered recipient.

The present invention also encompasses a pharmaceutical composition comprising an inhibitor of GPR132 wherein said inhibitor is a peptide or protein, a ribozyme, an antibody, a small molecule, an inhibitory RNA molecule, an antisense oligonucleotide, a morpholino or a lipid.

The term "pharmaceutical composition" as used herein refers to mixture comprising an inhibitor of GPR132 wherein said inhibitor is a peptide or protein, a ribozyme, an antibody, a small molecule, an inhibitory RNA molecule, an antisense oligonucleotide, a morpholino or alipid, in particular lysophosphatidylcholin (LPC). Moreover, the composition may comprise further components as well such as further therapeutic or auxiliary ingredients and/or pharmaceutically acceptable carriers and/or diluents. The pharmaceutical composition of the present invention is, preferably, to be used as a medicament. Said medicament is, preferably, applied to treat and/or prevent chemotherapy-induced neuropathic pain as described elsewhere herein in detail.

Preferably, such further ingredients of the pharmaceutical composition of the invention can be stabilizing agents, wetting agents, pharmaceutical carriers, additional pharmaceutically active agents, release controlling agents and the like. More preferably, the pharmaceutical composition of the present invention comprises a pharmaceutically acceptable carrier.

Preferred diluents encompass water, alcohols, physiological saline solutions, buffers, such as phosphate buffered saline solutions, syrup, oil, water, emulsions, various types of wetting agents, and the like. A pharmaceutically acceptable diluent according to the invention is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

A pharmaceutically acceptable carrier according to the invention must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatin, agar, pectin, acacia, magnesium stearate, stearic acid and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The pharmaceutical composition shall be adapted for use in treating and/or preventing chemotherapy-induced neuropathic pain. Typically, the desired mode of administration will be systemic administration. Thus, the composition of the invention is, preferably, formulated for a systemic application. Preferably, oral application, e.g. in the form of tablets, solution or drinking ampules, is envisaged or application via injection. However, depending on the nature and the mode of action, the composition may be administered by other routes as well including dermal, intra-muscular, subcutaneous, oral, intravenous or topical administration. The composition can be formulated for a bolus administration or can be made for continuous applications.

The composition of the invention shall, preferably, comprise the inhibitor of GPR132 referred to above in a therapeutically effective dose. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population) as describe above already.

For formulation as medicament, the composition of the present invention can be prepared in a manner well known in the pharmaceutical art. For example, for manufacturing a medicament, the therapeutically active ingredients will usually be mixed and, preferably, combined with a carrier or the diluent. The resulting formulations are to be adapted to the mode of administration. The procedures for formulating a medicament as referred to herein may involve mixing, granulating, compression or dissolving the ingredients as appropriate to form the desired composition. It will be appreciated that the form and character of the pharmaceutical acceptable carrier or diluent is dictated by the amount of active ingredient with which it is to be combined, the route of administration, and other well-known variables. Dosage recommendations shall be indicated in the prescribers or user's instructions in order to anticipate dose adjustments depending on the considered recipient. Finally, it is to be understood that the formulation of a composition according to the invention as a medicament takes place under GMP standardized conditions or the like in order to ensure quality, pharmaceutical security, and effectiveness of the medicament.

Said medicament is, preferably, applied to treat and/or prevent chemotherapy-induced neuropathic pain as described elsewhere herein in detail.

The definition and explanations given herein above apply mutatis mutandis to the following method of the present invention.

Moreover, encompassed by the present invention is a method, preferably an in vitro method, for identifying an inhibitor of GPR132 comprising the steps of
a) contacting a cell expressing GPR132 cultivated in the presence of an oxidized lipid with a compound suspected to be a GRP 132 inhibitor;
b) determining GPR132 activity after said contacting; and
c) comparing the determined GPR132 activity to GPR132 activity determined in a control cell, whereby an inhibitor of GPR132 is identified.

In a preferred embodiment, the aforementioned method is used for the identification of a medicament for the prevention and/or treatment of chemotherapy-induced neuropathic pain.

Accordingly, the present invention further relates a method, preferably an in vitro method, for the identification of a medicament for the prevention and/or treatment of chemotherapy-induced neuropathic pain, comprising the steps of
a) contacting a cell expressing GPR132 cultivated in the presence of an oxidized lipid with a compound suspected to be a GRP 132 inhibitor;
b) determining GPR132 activity after said contacting; and
c) comparing the determined GPR132 activity to GPR132 activity determined in a control cell, whereby a medicament for the prevention and/or treatment of chemotherapy-induced neuropathic pain is identified.

Further, the present invention relates to the use of the GPR132 polypeptide for identifying a medicament for the prevention and/or treatment of chemotherapy-induced neuropathic pain. Preferably, said use is an in vitro use.

Preferably, a compound is considered as an inhibitor of GPR132 and thus as a medicament for the prevention and/or treatment of chemotherapy-induced neuropathic pain, the GPR132 activity in step b) is lower than the GPR132 activity determined in a control cell.

It is to be understood that a cell expressing GPR132 can be a cell naturally expressing GPR132 or a cell that has been engineered to express GPR132 to a certain level, i.e. by introduction of a suitable expression construct. Such an expression construct can be, for example, a transiently or stably introduced recombinant DNA construct capable of expressing GPR132 in a constant and/or inducible manner. Preferably said cell expressing GPR132 is a mammalian cell, more preferably a mouse, monkey or human cell. Preferably, said cell is an immortal cell. Preferably, said cell is derived from monkey or human kidney tissue such as COS-1 or HEK293 cells. Preferably, said cell is stably expressing GPR132. Even more preferred, said cell is a HEK293 cell, stably expressing human GPR132 (NCBI Gene ID: 29933, Ensembl: ENSG00000183484 HPRD:06936).

Preferably, said recombinant DNA construct shall comprise an expression cassette for at least one polynucleotide encoding GPR132. In a preferred embodiment, the at least one polynucleotide encoding GPR132 present on the recombinant DNA construct, is stably integrated into the genome of a mammalian cell, more preferably a human cell. Means and methods for construction of a recombinant DNA construct are well known in the art and include, for example, polymerase chain reaction (PCR), use of restriction enzymes or gateway-cloning. Protocols to generate a recombinant DNA construct can also be found in standard text books such as Sambrook et al. (2001) Molecular Cloning: a laboratory manual, 3rd Edition, Cold Spring Harbor Laboratory Press, CSH, New York or Current Protocols in Molecular Biology, John Wiley & Sons, N.Y. (1989 and yearly updates). It will be understood that the recombinant DNA construct as referred to herein may contain additional regulatory elements such as an enhancer, a constitutive or inducible promoter or a terminator.

In a preferred embodiment, said recombinant DNA construct is based on the vector pcDNA3.1+ (Invitrogen). Preferably, the open reading frame of human GPR132 is amplified by PCR from human lung cDNA. Even more preferred, human GPR132 is cloned into pcDNA3.1+ (Invitrogen) using the restriction sites EcoRI(5') and XhoI(3'). It will be understood that the GPR132 polynucleotide comprised in said recombinant DNA construct may be the wild type polynucleotide (e.g. Gene Bank Accession Number: NM_013345) or may comprise one or more mutations. Preferably said one or more mutation is a silent mutation, i.e. a mutation in the polynucleotide sequence which does not cause a change in the polynuceotide sequence when translated. A respective DNA construct comprising the GPR132 polynuceotide with one silent mutation at nt138 C->A is also commercially available via cDNA.org (catalogue number #GPR1320000).

It will be understood, that said recombinant DNA construct may also encode for variants of GPR132. Such a variant, typically, differs from the specific amino acid sequence, e.g. from human GPR132 according to UniProtKB Accession Numbers Q9UNW8, by at least one amino acid substitution, deletion and/or addition. Nevertheless, such a variant of GPR132 shall still be capable of exerting the biological activity, in particular remaining the ability to bind to certain ligands and to activate a G-protein.

The term "polynucleotide" as used herein refers to a deoxyribonucleic acid or ribonucleic acid, for example to a single strand of a DNA polynucleotide or to a double stranded DNA polynucleotide. The length of a polynucleotide is designated by the specification of a number of basepairs ("bp") or nucleotides ("nt"). Also, as polynucleotides are defined by their respective nucleotide sequence, the terms nucleotide/polynucleotide and nucleotide sequence/polynucleotide sequence can be used interchangeably, thus that a reference to a nucleic acid sequence also is meant to define a nucleic acid comprising or consisting of a nucleic acid stretch, the sequence of which is identical to the nucleic acid sequence. Preferably, that polynucleotide to be used according to the present invention shall encode GPR132, more preferably human GPR132 (NCBI Gene ID: 29933, Ensembl: ENSG00000183484 HPRD:06936).

Said cell expressing GPR132 shall be cultivated in the presence of at least one (i.e. one or more than one) oxidized lipid. Accordingly, the medium in which the cell is cultivated shall comprise at least one oxidized lipid. Preferably, said oxidized lipid is selected from the group consisting of: hydroxyoctadecadienoic acids (HODEs), dihydroxyoctadecenoic acids (DiHOMEs) and epoxyoctadecenoic acids (EpOMEs). More preferably, said oxidized lipid is selected from the group consisting of 9-HODE, 13-HODE, 9,10-DiHOME, 12,13-DiHOME, 9,10-EpOME and 12,13-EpOME. Most preferably said lipid is 9-HODE.

Preferably, the concentration of said at least one oxidized lipid the medium in which the cell is cultivated is at least 100 nM. More preferably, the concentration is in the range of 100nM to 10µM. Even more preferably, the concentration of said at least one oxidized lipid the medium in which the cell is cultivated is in the range of about 5µM to 10µM, 2µM to 5µM, 100nM to 2µM, 500nM to 2mM, 500 nM to 1µM. Most preferably the concentration is about 1µM.

About as referred to herein refers to any specific value referred to in this specification, e.g., the concentration of an oxidized lipid in the medium, including any variation which is within the range of+/-20%, +/-10%, +/- 5%, +/-4%, +/-3%, +/-2% or +/-1%.

A compound suspected to be a GRP132 inhibitor can be any compound suspected to be capable of partially or fully impairing the activity of GPR132. Inhibition of GPR132 activity can be achieved by direct or indirect impairment of GPR132 activity as described elsewhere herein in more detail. Preferably, said compound suspected to be a GRP132 inhibitor is a peptide or protein, a ribozyme, an antibody, a small molecule, an inhibitory RNA molecule, an antisense oligonucleotide, a morpholino, a lipid, in particular lysophosphatidylcholin (LPC).

Contacting a cell expressing GPR132 with a compound suspected to be a GRP132 inhibitor is performed such that the compound is potentially capable of partially or fully impairing the activity of GPR132. For example, the compound suspected to be a GRP132 inhibitor can be applied to the supernatant of a cell expressing GPR132 or directly induced or expressed within a cell expressing GPR132.

Determining GPR132 activity, preferably, refers to the measurement of a signal indicative of GPR132 activity. As described above, GPR132 is a receptor that is coupled to a G-protein. Once GPR132 is activated, for example by 9-HODE, the activated G-protein (bound to GTP) induced further signaling events, leading to activation of protein kinases such as PLC and/or PLK and subsequent calcium release from the endoplasmic reticulum. Accordingly, the signal indicative of GPR132 activity is, preferably, the release of calcium. Thus, the activity of GPR132 is preferably determined by assessing the calcium release effected by the GPR132 polypeptide. Means and methods to determine the activity of a G-protein coupled receptor, including GPR132, are well known in the art and include, for example, measuring conformational change in the G-protein coupled receptor and/or subsequent activation of downstream signaling molecules as also described above.

Preferably, the GPR132 activity is determined by measuring calcium release (i.e. the release which is effected by the GPR132 polypeptide. Said calcium release is preferably measured by fluorescent calcium imaging. More preferably, said GPR132 activity is determined by using Fura-2, which is a ratiometric fluorescent dye that binds to free intracellular calcium and/or by using aequorin which is a calcium-activated photoprotein isolated from the hydrozoan Aequorea victoria. Fura-2 is a membrane permeable dye and protocols to measure calcium using Fura-2 are well described in the art. Typically, the excitation ratiometric imaging of Fura-2 is carried out by using 340/26 and 380/10 excitation filters, a 455 nm dichroic mirror (with good reflectivity down to approximately 330 nm), and a 535/40 emission filter. Aequorin is a bioluminescent protein that by itself typically generates blue light, the emitted light can easily be detected, for example using a luminometer. Aequorin can also be used together with the Green fluorescent protein (GFP) to produce green light by resonant energy transfer. For determining GPR132 activity using aequeorin, aequeorin is preferably expressed with the cell using a recombinant DNA construct. Means and methods for generating a recombinant DNA construct are well known in the art and also described elsewhere herein. More preferably, aequorin is co-expressed in the cell stably or transiently expressing GPR132.

The term "comparing" as used herein refers to comparing the GPR132 activity in a cell that has been contacted with a compound suspected to be a GRP132 inhibitor with a control cell that has not been contacted with a compound suspected to be a GRP132 inhibitor. It is to be understood that comparing as used herein usually refers to a comparison of corresponding parameters or values, e.g., an absolute amount is compared to an absolute reference amount while a concentration is compared to a reference concentration or an intensity signal obtained from the activation of GPR132 in a cell is compared to the same type of intensity signal obtained from a control cell. Such an intensity signal may be the amount of emitted light by a fluorescent or bioluminescent molecule, for example the fluorescence emitted by Fura-2 and/or Aequorin as described elsewhere herein. The comparison may be carried out manually or computer assisted. Thus, the comparison may be carried out by a computing device. For example, the intensity signal obtained from the activation of GPR132 in a cell and a control cell, respectively, can be, e.g., compared to each other and the said comparison can be automatically carried out by a computer program executing an algorithm for the comparison. The computer program carrying out the said evaluation will provide the desired assessment in a suitable output format. For a computer assisted comparison, the value the intensity signal obtained from the cell and the control cell or the calculated ration of the two values, respectively, is stored in a database by a computer program. The computer program may further evaluate the result of the comparison, i.e. automatically provide the desired assessment in a suitable output format. A compound suspected to be an inhibitor of GPR132 that causes a lower signal indicative of GPR132 activity in the cell that has received said compound compared to the control cell that has not received said compound, is regarded as an inhibitor of GPR132. Preferably, the signal in the cell that has received an inhibitor of GPR132 is decreased by at least 10%, more preferably by at least 20% and even, more preferably, by at least 30%, and most preferably by at least 40%.

The term "control cell" refers to a cell, preferably a mammalian, more preferably a mouse or human cell, which shows GPR132 activity. Preferably, a control cell is a cell expressing GPR132 cultivated in the presence of an oxidized lipid which has not been contacted with a compound suspected to be a GRP132 inhibitor. Preferably, the control cell as well as the cell that is contacted with a compound suspected to be a GRP132 inhibitor belong to the same cell type.

More generally, the present invention also contemplates the use of an inhibitor of GPR132 for the manufacture of a medicament for the prevention and/or treatment of chemotherapy-induced neuropathic pain in a subject, wherein said subject has received at least one platinum-containing chemotherapeutic agent.

Another general aspect of the present invention relates to a method for prevention and/or treatment of chemotherapy-induced neuropathic pain in a subject in need thereof comprising administering to a subject an inhibitor of GPR132, wherein said subject has received at least one platinum-containing chemotherapeutic agent, Preferably, said inhibitor is administered to the subject in a pharmaceutically effective amount.

Advantageously, it has been found in accordance with the studies underlying the present invention that inhibition of the G-protein coupled receptor 132 (GPR132), also named G-protein coupled receptor G2A (G2A receptor), leads to significant reduction of chemotherapy -induced peripheral neuropathic pain (CIPN) induced by platinum-containing chemotherapeutic agents, and especially by oxaliplatin. Moreover, the lipid ligand of GPR132, 9-HODE (9-hydroxy-10E,12Z-octadecadienoic acid), was found in increased concentrations in sciatic nerves and dorsal root ganglia of oxaliplatin treated mice. This lipid sensitizes the ion channel TRPV1 in sensory neurons via activation of GPR132 and PKC. Mice that are deficient of the GPR132-receptor show deceased thermal and/or mechanical allodynia after oxaliplatin treatment. Inhibitors of GPR132 can be used to reduce TRPV1-sensitization and hyperexcitability of sensory neurons and thus to reduce oxaliplatin-induced neuropathic pain in patients.

The above explanations and definitions of the terms apply throughout the specification. Moreover, in the following, typical embodiments of the composition according to the present invention are listed.

In a preferred embodiment of the inhibitor of GPR132 for use, the at least one platinum-containing chemotherapeutic agent is oxaliplatin.

In yet a preferred embodiment of the inhibitor of GPR132 for use, the inhibitor reduces chemotherapy-dependent hyper-excitability of sensory neurons.

In yet a preferred embodiment of the inhibitor of GPR132 for use, the inhibitor reduces chemotherapy-dependent sensitization of a transient receptor potential (TRP) channel.

In yet a preferred embodiment of the inhibitor of GPR132 for use, the transient receptor potential (TRP) channel is selected from the group consisting of: TRPV1, TRPA1, TRPM8, TRPV4, TRPC3 and TRPC6.

In yet a preferred embodiment of the inhibitor of GPR132 for use, the chemotherapy-dependent sensitization of a transient receptor potential (TRP) channel is mediated by oxidized lipids.

In yet a preferred embodiment of the inhibitor of GPR132 for use, the oxidized lipids are selected from the group consisting of: hydroxyoctadecadienoic acids (HODEs), dihydroxyoctadecenoic acids (DiHOMEs) and epoxyoctadecenoic acids (EpOMEs).

In yet a preferred embodiment of the inhibitor of GPR132 for use, the oxidized lipids are selected from the group consisting of: 9-HODE, 13-HODE, 9,10-DiHOME, 12,13-DiHOME, 9,10-EpOME and 12,13-EpOME.

In yet a preferred embodiment of the inhibitor of GPR132 for use, the subject is a mammal, preferably a human.

In yet a preferred embodiment of the inhibitor of GPR132 for use, the inhibitor is given to said subject by oral and/or parental administration.

In yet a preferred embodiment of the inhibitor of GPR132 for use, the inhibitor is a peptide or protein, a ribozyme, an antibody, a small molecule, an inhibitory RNA molecule, an antisense oligonucleotide, a morpholino or a lipid, in particular lysophosphatidylcholine (LPC).

It will be understood that the present invention also provides a pharmaceutical composition comprising an inhibitor of GPR132 wherein said inhibitor is a peptide or protein, a ribozyme, an antibody, a small molecule, an inhibitory RNA molecule, an antisense oligonucleotide, a morpholino or a lipid, in particular lysophosphatidylcholine (LPC).

In a preferred embodiment of the pharmaceutical composition of the present invention, the pharmaceutical composition further comprises a pharmaceutically acceptable carrier.

Moreover, also encompassed by the present invention is a method for identifying an inhibitor of GPR132 comprising the steps of
a) contacting a cell expressing GPR132 cultivated in the presence of an oxidized lipid with a compound suspected to be a GRP 132 inhibitor;
b) determining GPR132 activity after said contacting; and
c) comparing the determined GPR132 activity to GPR132 activity determined in a control cell, whereby an inhibitor of GPR132 is identified.

In a preferred embodiment of the method, the control cell is a cell expressing GPR132 cultivated in the presence of an oxidized lipid which has not been contacted to a compound suspected to be a GRP 132 inhibitor.

All references cited in this specification are herewith incorporated by reference with respect to their entire disclosure content and the disclosure content specifically mentioned in this specification. Full citations of the references are to be found elsewhere herein.

### FIGURES

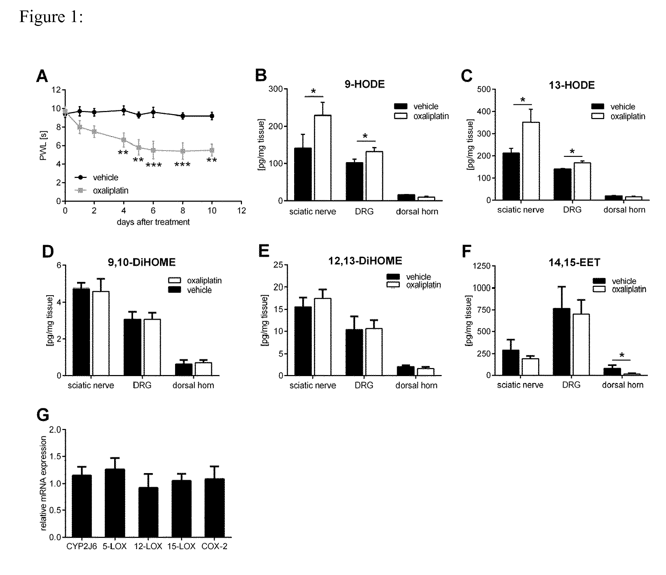

**Figure 1****: Increased synthesis of 9-HODE and 13-HODE in sciatic nerve and DRGs after 10 days treatment with oxaliplatin.** A: Mechanical pain thresholds of mice after i.p.-injection of oxaliplatin (3 mg/kg) compared to vehicle (0.9 % v/v DMSO) using a dynamic plantar aesthesiometer over 10 days. Shown is the mean ± SEM of paw withdrawal latency (PWL) of n=8 mice per group; **p<0.01 ***p<0.001; two-way ANOVA with Bonferroni post-hoc test. B-F: Concentrations of 9-HODE (B), 13-HODE (C), 9,10-DiHOME (D), 12,13-DiHOME (E) and 14,15-EET (F) from sciatic nerve, DRGs and dorsal horn tissue of mice treated with vehicle (black bars; 0.9 % v/v DMSO) or oxaliplatin (white bars; 3 mg/kg) 10 days after injection. Data represent mean ± SEM from n=5 mice per group; *p<0.05; unpaired student's t-test. G: Relative mRNA expression of CYP2J6, COX2 and LOX-isoforms in dorsal root ganglia (DRGs) from mice 10 days post treatment with oxaliplatin (3 mg/kg) compared to vehicle (0.9 % v/v DMSO) using quantitative RT-PCR. Data represent mean ± SEM from n=3 mice per group.

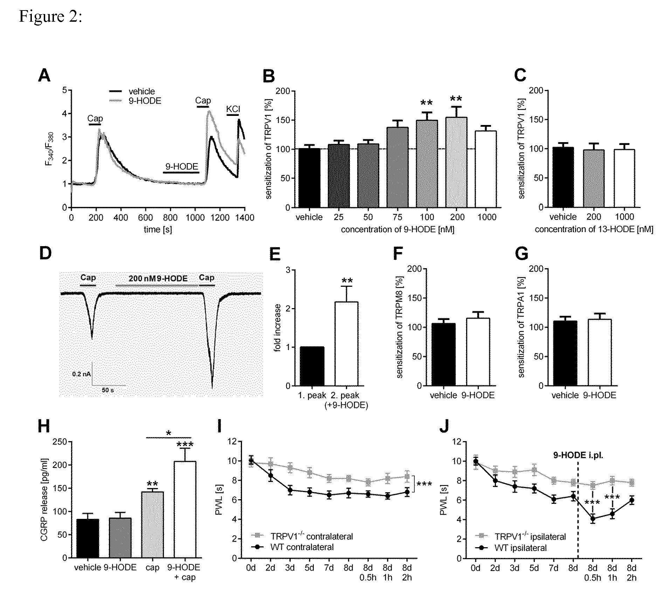

**Figure 2****: 9-HODE sensitizes TRPV1 in sensory neurons and reduces mechanical pain threshold in vivo.** A: Stimulation of TRPV1 of adult DRG neurons two times with capsaicin (Cap; 200 nM, 30 s). Incubation with 9-HODE (200 nM, 120 s) before the second stimulus leads to an increased calcium flux compared to the first stimulus (Δratio). Neurons were identified by responses to KCl (40 mM, 10 s). Shown is a representative trace. B: 9-HODE dose-dependently sensitizes the TRPV1 channel of DRG neurons. Shown is the Δratio of the first and second capsaicin stimulus at 9-HODE concentrations ranging from 0 to 1000 nM. Data are presented as mean ± SEM with n=44-99 neurons per concentration. C: 13-HODE (200 nM, 120 s) does not increase the Δratio of TRPV1-dependent calcium flux in DRG neurons (n=30-83). D: Sample trace of whole-cell voltage clamp measurement of DRG neurons treated with capsaicin (1 µM, 15s) twice. Before the second cap-stimulus, cells were incubated with 9-HODE (200 nM, 3 min). E: Normalized response to first and second capsaicin-stimulus with cells treated like in (D) shown as mean ± SEM with n=11 neurons. Effect of 9-HODE on TRPM8 (F) of DRG neurons stimulated with menthol (100 µM, 30 s) and TRPA1 (G) stimulated with allyl isothiocyanate (50 µM, 30 s). Seen is the Δratio compared to vehicle (DMSO) with n=20-33 neurons per condition. H: CGRP release from DRG neurons stimulated with 9-HODE (1 µM, 10 min), capsaicin (400 nM, 10 min) or both together. Data shown as mean ± SEM from n=6-8 mice per condition; *p<0.05 **p<0.01 ***p<0.001; unpaired student's t-test. I: Mechanical pain thresholds of wildtype mice (WT) compared to TRPV1-deficient mice after treatment with oxaliplatin (3 mg/kg) using a dynamic plantar aesthesiometer over 8 days. Shown is the mean ± SEM of PWL of the contralateral paw (untreated) of n=7 mice per group; ***p<0.001; two-way ANOVA. J: Mechanical pain thresholds of wildtype mice compared to TRPV1-deficient mice after treatment with oxaliplatin (3 mg/kg). At day 8, 9-HODE (1 µM) was injected i.pl. (intraplantar) into the ipsilateral paw and PWL was measured again after 0.5 h, 1 h and 2 h. Shown is the mean ± SEM of PWL of the ipsilateral paw (9-HODE treated) of n=7 mice per group; ***p<0.001; two-way ANOVA with Bonferroni post-hoc test.

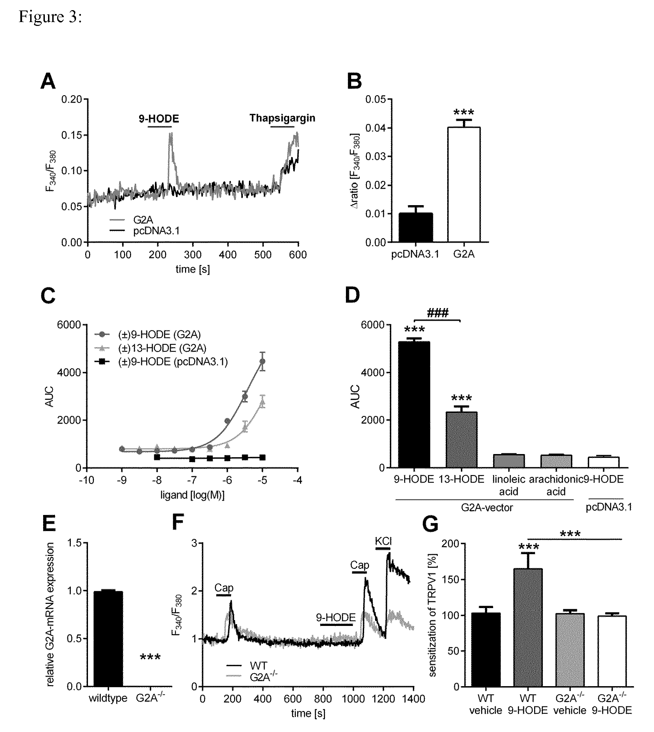

**Figure 3****: 9-HODE activates the G2A-receptor** (**GRP132**). A-B: Shown are calcium concentrations of HEK-cells transfected with G2A-vector or pcDNA3.1 (empty vector) stimulated with 9-HODE (1 µM). Thapsigargin was used as positive control. A shows a representative trace and B the quantitative analysis presented as mean ± SEM with n=32-46 cells per condition. ***p<0.001; unpaired student's t-test. C: Dose-response analysis of COS-1 cells cotransfected with aequorin- and G2A-expressing vector or pcDNA3.1 (empty vector). Seen is the calcium flux as arear under curve (AUC) at different 9-HODE and 13-HODE concentrations [10-9 - 10-5 M]. Data are presented as mean ± SEM from n=3 measurements. D: COS-1 cells cotransfected with aequorin- and G2A-expressing vector stimulated with 9-HODE (10 µM), 13-HODE (10 µM), linoleic acid (30 µM) and arachidonic acid (30 µM). Shown is the calcium flux as mean of arear under curve (AUC) ± SEM with n=3 measurements; ***p<0.001; One-way ANOVA with Bonferroni post-hoc test; ###p<0.001 unpaired student's t-test. E: Expression of G2A-mRNA in wildtype and G2A-deficient mice. Data are presented as mean ± SEM with n=4 measurements; ***p<0.001; unpaired student's t-test. F-G: Incubation of adult DRG neurons with 9-HODE (200 nM, 120s) prior to the second capsaicin stimulus (Cap; 200 nM, 30 s) leads to an increased calcium flux compared to the first stimulus (Δratio) in DRG neurons of wildtype (WT) but not in G2A-deficient mice. Neurons were identified by responses to KCl (40 mM, 10 s). F Shows a representative trace and G the quantitative analysis presented as mean ± SEM with n=46-110 cells per condition; ***p<0.001; One-way ANOVA with Bonferroni post-hoc test.

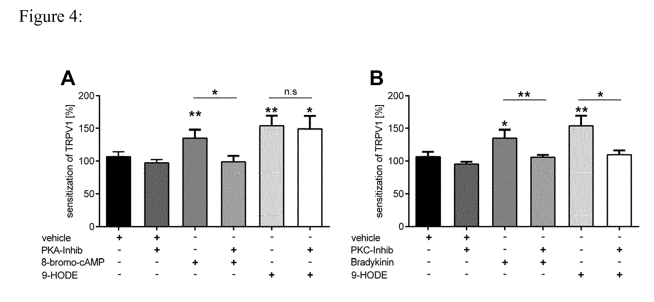

**Figure 4****: TR-PV1-sensitization via 9-HODE is mediated by PKC but not PKA.** A: Sensitization of TRPV1 via 9-HODE (200 nM, 120s) of adult DRG neurons is not influenced by the PKA-Inhibitor H89 (10µM, 45 mins before measurement). As control for PKA-dependent TRPV1 sensitization 8-bromo-cAMP (40 µM, 120 s) was used. Data shown as mean ± SEM with n=20-69 cells per condition. B: The PKC-Inhibitor GF109203X (1 µM, 45 mins before measurement) supresses the 9-HODE-dependet sensitization of TRPV1. Bradykinin (500 nM, 120 s) was used as positive control for PKC-dependent sensitization of TRPV1. Data is shown as mean ± SEM with n=45-90 cells per condition; *p<0.05 **p<0.01; unpaired student's t-test.

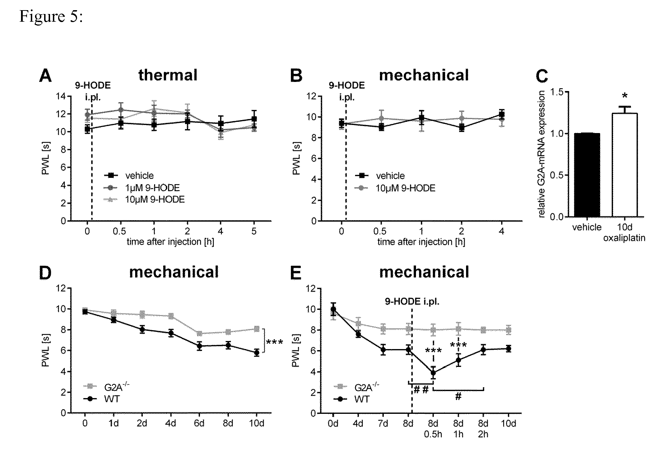

**Figure 5****: G2A-deficient mice have reduced 9-HODE-dependent mechanical pain during oxaliplatin treatment.** A: Thermal pain thresholds of naive wildtype mice after i.pl. (intraplantar) injection of 9-HODE (1 µM and 10 µM) into the left hind paw using a Hargreaves Apparatus. Shown is the mean ± SEM of PWL of n=8 mice per group B: Mechanical pain thresholds of naive wildtype mice after i.pl. injection of 9-HODE (10 µM) into the left hind paw using a dynamic plantar aesthesiometer. Shown is the mean ± SEM of PWL of n=6 mice per group. C: G2A-mRNA expression in DRG of mice treated with oxaliplatin (3 mg/kg) for 10 days compared to vehicle treated mice. Data presented as mean ± SEM with n=4 mice per group; *p<0.05; unpaired student's t-test. D: Mechanical pain thresholds of wildtype (WT) compared to G2A-deficient mice after treatment with oxaliplatin (3 mg/kg) using a dynamic plantar aesthesiometer over 10 days. Shown is the mean ± SEM of PWL of n=8 mice per group; ***p<0.001; two-way ANOVA. E: Mechanical pain thresholds of wildtype compared to G2A-deficient mice after treatment with oxaliplatin (3 mg/kg) over 8 days. Then 9-HODE (10 µM) was injected i.pl. (intraplantar) into the left hind paw and PWL was measured again for 0.5 h, 1 h and 2 h. Shown is the mean ± SEM of PWL of the 9-HODE treated paw of n=7 mice per group; ***p<0.001, two-way ANOVA with Bonferroni post-hoc test; ##p<0.01, unpaired student's t-test.

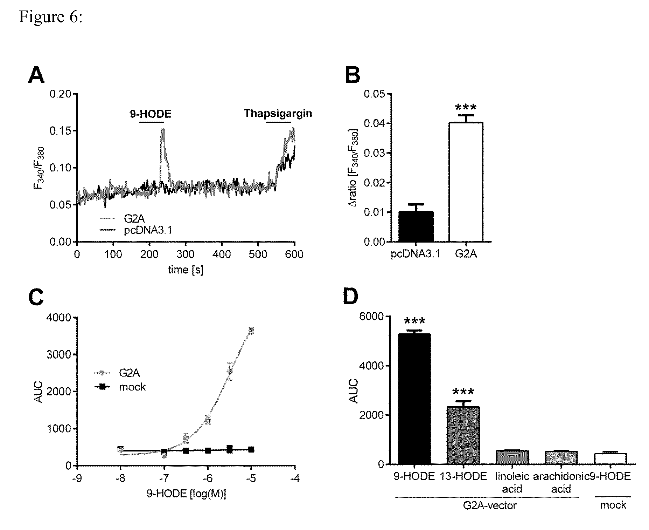

**Figure 6****: Determination of GPR132 activity in a cell based assay.** (A) and (B): HEK-293 cells were transiently transfected with GPR132. Activation of GPR132 by 9-HODE (1 µM) caused calcium-release. Calcium-readout: fluorescence intensity of FURA-2. (C) and (D): COS-1-cells were transiently transfected with GPR132 and co-transfected with a vector expressing Aequorin. Activation of GPR132 by 9-HODE caused calcium release in a concentration-dependent manner (10 nM - 10 µM). Calcium-readout: Bioluminescence of Aequorin.

### EXAMPLES

The invention will be merely illustrated by the following Examples. The said Examples shall, whatsoever, not be construed in a manner limiting the scope of the invention.

### Example 1: Determination of Lipids from Tissue Samples by LC-MS/MS

The spinal cord from L4-L6, both sciatic nerves and twenty to thirty dorsal root ganglia (DRGs) were prepared from adult mice.

### Lipid extraction and standards

Stock solutions with 2500 ng/ml of the analytes 9-HODE, 13-HODE, 9,1 0-EpOME, 12,13-EpOME, 9,10-DiHOME, 12,13-DiHOME, 5,6-EET, 8,9-EET, 11,12-EET and 14, 15-EET as well as their internal standards (9-HODE-d4, 13-HODE-d4, 9,10- and 12, 13-EpOME-d4, 9,10- and 12, 13-DiHOME-d4, 5,6- and 14, 15-EET-d11, 8,9- and 11, 12-EET-d4) were prepared in methanol. Working standards were obtained by further dilution with a concentration range of 0.25-2500 ng/ml for the analyte.

Sample pre-treatment was performed using liquid-liquid extraction. Therefore, homogenated tissue was extracted twice with 600 ~I of ethyl acetate. The combined organic phases were removed at a temperature of 45°C under a gentle stream of nitrogen. The residues were reconstituted with 50 ~I of methanol/water/butylated hydroxytoluene (BHT) (50:50:10-3, v/v/v) and then centrifuged for 2 min at 10,000 g, and transferred to glass vials waiting for analysis.

### Instrumentation for lipid measurement

The LC-MS/MS system consisted of a QTrap 5500 (Sciex, Darmstadt, Germany) equipped with a Turbo-V source operating in negative electrospray ionization mode, an Agilent 1200 binary HPLC pump and degasser (Agilent, Waldbronn, Germany), and an HTC Pal autosampler (CTC analytics, Zwingen, Switzerland). High-purity nitrogen for the mass spectrometer was produced by a NGM 22-LC-MS nitrogen generator (erne Instruments, Eschborn, Germany).

For the chromatographic separation of oxidized lipids, a Gemini NX C18 column and precolumn were used (150x2 mm inner diameter, 5 µm particle size, and 110 A pore size; Phenomenex, Aschaffenburg, Germany). A linear gradient was used at a flow rate of 0.5 ml/min with a total run time of 17.5 min. Mobile phase A consist of water:ammonia (100:0.05, v/v), and mobile phase B of acetonitrile:ammonia (100:0.05, v/v). The gradient changed from 85% A to 10% within 12 min. These conditions were held for 1 min. Then, the mobile phase shifted back to 85% A within 0.5 m in and it was maintained for 4 min to re-equilibrate the column.

20 µl of the extracted samples were injected into the LC-MSIMS system. Quantification was performed with MultiQuant 3.0 (Sciex, Darmstadt, Germany) using the internal standard method (isotope-dilution mass spectrometry). Ratios of analyte peak area and internal standard area (y-axis) were plotted against concentration (x-axis), and calibration curves were calculated by least-squares regression with 1/square concentration weighting.

### Example 2: Calcium measurements, Neuronal calcitonin gene-related peptide release and Electrophysiology recordings

### DRG primary cultures

DRG primary cultures were prepared as described previously (Brenneis et al., 2011 b). Briefly, twenty to thirty murine dorsal roots ganglia (DRGs) were dissected from spinal segments and transferred directly into ice-cold Hanks' balanced salt solution with CaCI2 and MgCI2 (lnvitrogen, Carlsbad, CA, USA). The DRGs were than incubated with Neurobasal Medium (lnvitrogen, Carlsbad, CA, USA) containing 500 U/ml collagenase (Sigma, Deisenhofen, Germany) and 2.5 U/ml dispase II (Roche, Mannheim, Germany) for 75 mins at 37 °C. After removal of the collagenase/dispase-solution cells were washed three times with medium containing 10 % FCS, penicillin / streptomycin / gentamycin (lnvitrogen, Carlsbad, CA, USA) and incubated in 0.05 % trypsin for 10 min at 37 °C. After two washing steps, the cells were mechanically dissociated with a 1 ml pipette and plated on Poly-L-Iysine (Sigma) coated coverslips. After incubation at 37°C for 2h, Neurobasal Medium containing glutamine (2 mm), penicillin (100 U/ml), streptomycin (100 µg/ml), B-27 and gentamicin (50 µg/ml) was added to the cells and incubated overnight. The neurons were used for Calcium imaging the next day.

### Calcium Imaging

Calcium-imaging experiments were performed with a Leica calcium-imaging setup, consisting of a Leica DMI 4000 b inverted microscope equipped with a DFC360 FX (CCD) camera, Fura-2 filters, and an N-Plan 10x/0.25 Phl objective (all from Leica), was used. Images were taken every 2 s and were processed with the LAS AF-software (Leica). For each experiment, an area with large cell numbers 24h after preparation (see DRG primary cultures) was chosen and 40-110 cells were monitored simultaneously. Cells were loaded with 5 µM fura-2-AM-ester (Biotium), incubated for 45-60 min at 37 °C and washed with ringer solution (145 mM NaCI, 1.25 mM CaCl₂, 1 mM MgCl₂, 5 mM KCI, 10 mM D-glucose, and 10 mM HEPES, adjusted to pH 7.3). Baseline measurements were performed with ringer solution at a flow rate of 1-2 ml/min at room temperature. For the activation of TRPV1 we used a solution of 200 nM capsaicin (Sigma), for TRPA1 activation 50 µM allyl isothiocyanate (AITC, Fluka Analytic) and for TRPM8 100 µM L-menthol (Sigma) dissolved in ringer solution. For TRP-channel sensitization, cells were incubated for 2 mins with different concentrations (50 nM - 1000 nM) of(±) 9-HODE (Cayman) or (±) 13-HODE (Cayman) before the second stimulus took place. For control stimulations, the corresponding volume of DMSO (Sigma) was mixed in ringer solution and applied with the same flow rate. 10 µM of the PKA inhibitor H89 (Tocris) or 1 µM of the PKC inhibitor GF1 09203X (Tocris) was co-incubated with fura-2 for 45 mins. As positive control for PKA activation 40 iJM 8-bromo-cAMP (Cayman) and for PKC activation 500 nM bradykinin (Cayman) was used. At the end of each measurement, 50 mM KCI was applied to identify neurons.

### Neuronal calcitonin gene-related peptide release

For the determination of neuronal calcitonin gene-related peptide (CGRP) release, dorsal root ganglia (DRGs) neurons were isolated from mice as previously described (Brenneis et al., 2011 a). Briefly, DRG cultures were stimulated for 10 m in with 1 µM (±) 9-HODE (Cayman) alone or in combination with 400 nM capsaicin (Sigma). Cultures supplemented with capsaicin alone served as control. CGRP concentrations in supernatants were analysed utilizing the rat CGRP Enzyme ImmunoAssay Kit (SPI-Bio, Montigny, France) according to the manufacturer's instructions. Absorption was measured with the help of the Spectra Max 250 (Molecular Devices, lsmaning, Germany).

### Electrophysiology

All recordings were carried out at room temperature within 48 hour of plating DRG neurons. Whole-cell voltage clamp recordings were made with an Axopatch 200A amplifier (Molecular Devices) and patch pipettes with resistances of 2-3 MΩ. Pipette solution was 5 mM NaCl, 140 mM KCl, 0.5 mM CaCl2, 2 mM Macl2, 5 mM EGTA, 10 mM HEPES, and 3 mM Na2ATP, pH 7.2, adjusted with NaOH. The external solution was 140 mM NaCl, 5 mM KCl, 2 mM CaCl2, 2 mM MgCl2, 10 mM HEPES, and 10 mM D-glucose, pH 7.4, adjusted with NaOH (Sigma). Command protocols were generated and data was digitized with a Digidata 1440A A/D interface with pCLAMP10 software. We used gravity perfusion system connected with perfusion pencil (AutoMate Scientific) to externally apply 9-HODE or capsaicin to the cells.

### Calcium measurement in vitro

For studies of heterologously expressed G2A receptor, COS-1 cells were seeded in white walls-clear bottom 96-well plates and transfected with a plasmid encoding a calcium-sensitive bioluminescent fusion protein between aequorin and GFP (Baubet et al., 2000) together with plasmid containing cDNA for G2A or control DNA (empty vector, mock) at a concentration of 50 ng/well by using FuGENE 6 reagent (Promega) following manufacturer's instructions. Forty eight hours later, cells were loaded with 5 µM coelenterazine h (Invitrogen) in HBSS containing 1.8 mM CaCl2 and 10 mM glucose for 2h at 37°C. Measurements were performed by using a luminometric plate reader (Flexstation 3). The area under each calcium transient was calculated by using SoftMaxPro software and expressed as area under the curve (AUC).

### Example 3: Behavioral tests in mice

### Animals

In all behavioral tests the experimenter was unaware of the treatment and the genotype of the mice. For all behavioral experiments we used only 6-12 weeks old C57BU6N mice purchased from commercial breeding companies (Charles River, Sulzfeld, Germany, Janvier, Le Geneset-Saint-lsle, FR). G2A and TRPV1 knockout strains were from Jackson laboratories. All experiments followed the ethics guidelines for investigations in conscious animals and the procedures were approved by the local Ethics Committee.

### Treatments

For the intra plantar (i.pl.) treatment with 9-HODE (1 0 (JM) (Cayman), a volume of 10 (JI was injected in the mid-plantar area of the hindpaw. For the intraperitoneal (i.p.) treatment, 100 (JI of oxaliplatin (3 mg/kg,Tocris) were injection into the peritoneum. The control group animals were treated with the corresponding volumes of 0.9% saline solution.

### Behavioral Testing - Mechanical pain

To determine the mechanical thresholds of mice, a Dynamic Plantar Aesthesiometer (Ugo Basile, Comerio, Italy) was used. Mice were put on an elevated grid in test cages at least 2h before the measurement to allow accommodation. Then, baseline measurement was performed before the treatment with Oxaliplatin or 9-HODE. Therefore, a steel rod pushed against the plantar side of the hind paw with linear ascending force (0-5 g over 10 s, in 0.5 g/s intervals) until a fast withdraw of the paw was observed The time between the first contact of the steel rod and the paw withdraw was measured in seconds (paw withdraw latency) and a cut-off time of 20 s was set.

### Behavioral Testing - Thermal pain

To determine the thermal thresholds, mice were kept in test cages on a warmed glass plate (32 °C) for at least 2h on the first day to allow accommodation. Then the mid-plantar region of the paws was stimulated with a radiant heat device, consisting of a high-intensity projector lamp, until withdrawal occurred. The non-injected and injected paws were measured alternately in intervals of 5-10 min.

### Example 4: Real-time RT-PCR, Data Analysis and Statistics

### Real-time RT-PCR

Lumbar DRGs (L2-L6) were dissected, quick-frozen in liquid nitrogen and stored at -80 °C. Total RNA of the DRGs was isolated under RNase-free conditions using a mirVana miRNA Isolation Kit (Ambion) according to the manufacturer's instructions and quantified with a NanoDrop ND-1000 spectrophotometer (NanoDrop Technologies). The cDNA was synthesized from 200 ng RNA using a First Strand cDNA Synthesis Kit (Thermo Scientific) according to the manufacturer's instructions. Real-time RT-PCR was performed using an ABI Prism 7500 Sequence Detection System (Applied Biosystems) using Maxima SYBR Green qPCR Master Mix ROX as well as suitable primers for CYP2C37 (Luo et al., 1998), CYP2C38 (Zhang et al., 2003), CYP2C39 (Luo et al., 1998), CYP2J6 (Choudhary et al., 2003) and CYP3A11 (Chavan et al., 2015).

For analysis of GAPDH (Mm99999915_g1), G2A (Mm02620285_s1), 5-LOX (Mm01182747 m1), 12-LOX (Mm00545833_m1), 15-LOX (Mm00507789_m1) and COX-2 (Mm03294838_g1) assay primer from ThermoFisher Scientific were used. Reactions (total volume 10 µl) were performed in triplicate by incubating at 95 °C for 10 min, followed by 40 cycles of 15 s at 95 °C and 1 min at 60 °C. Water controls were included to ensure specificity. Relative expression of target gene levels was determined using the ΔΔCt method, with Ct indicating the cycle number at which the signal of the PCR product crosses a defined threshold set within the exponential phase of the PCR. The amount of sample RNA was normalized to GAPDH.

### Data Analysis and Statistics

All data are presented as mean ± SEM. To determine statistically significant differences in all behavioral experiments, Two-way ANOVA for repeated measures was used, followed by Bonferroni's *post hoc* correction using GraphPad Prism 6. For *in vitro* experiments comparing only *two* groups, Student's *t test* was carried out. A p value of< 0.05 was considered statistically significant.

### Example 5: Increased synthesis of oxidized lipids in sciatic nerve and DRGs of oxaliplatin treated mice

To investigate whether or not alterations in concentrations of oxidized lipids occur during oxaliplatin-induced neuropathic pain, 3 mg/kg oxaliplatin (Nassini et al., 2011) was injected i.p. in C57BL/6N mice and the paw withdrawal latency over 10 days was monitored using a dynamic plantar aesthesiometer. Treated mice showed significantly reduced mechanical paw withdrawal latencies starting at day 4 with maximal effects between day 8 to 10 (Fig. 1A). Sciatic nerves, the L1-L6 DRGs and the corresponding section of the dorsal horn were dissected 10 days after oxaliplatin injection from mice and the concentration of oxidized lipids was determined by LC-MS/MS. The concentrations of hydroxyoctadecadienoic acids (HODEs), dihydroxyoctadecenoic acids (DiHOMEs), epoxyoctadecenoic acids (EpOMEs) and epoxyeicosatrienoic acids (EETs) were analyzed. 9-HODE (Figs. 1B-F). To assess the expression regulation of enzymes synthesizing oxidized lipids, the mRNA levels of the cytochrome-P450-(CYP)-epoxygenase CYP2J6, the lipoxygenases isoforms (LOX), 5-LOX, 12-LOX, 15-LOX as well as cyclooxygenase-2 (COX-2) were determined in lumbal DRGs (Figure 1G). No difference in mRNA expression of these enzymes at day 10 after oxaliplatin treatment was observed.

### Example 6: 9-HODE sensitizes TRPV1 in sensory neurons and reduces mechanical pain thresholds in vivo

To investigate whether or not 9-HODE or 13-HODE can sensitize TRPV1, DRG neurons from mice were stimulated with capsaicin and incubated with vehicle, 9-HODE or 13-HODE for 2 minutes before a second capsaicin stimulus was applied (Figure 2A). The Δratio of the first and second peak was calculated to determine whether or not a sensitization towards capsaicin stimulation occurred (Figure 2B). 9-HODE concentration-dependently induced a sensitization of TRPV1 at concentrations from 100 nM to 200 nM of 9-HODE (Figure 2B). In contrast, 13-HODE had no strong sensitizing effect on capsaicin-induced TRPV1 activation (Figure 2C). Whole-cell voltage clamp measurement of DRG neurons confirmed that 9-HODE induces TRPV1 sensitization, since the capsaicin-induced inward current of cells pretreated with 9-HODE was significantly increased (Figure 2D and Figure 2E). Fittingly, 9-HODE increased the capsaicin-dependent CGRP release significantly in DRG neurons (Figure 2H). Notably, TRPV1 sensitization by 9-HODE was specific for TRPV1, since 9-HODE showed no sensitizing effects for TRPM8 (activated with menthol [100 µM]; Figure 2F) or TRPA1 (activated with AITC [50 µM]; Figure 2G). TRPV1-deficient mice displayed reduced mechanical paw withdrawal latencies over the complete time period of 8 days after treatment with oxaliplatin compared with wild type mice (Figure 2I). Furthermore, intraplantar injection of 9-HODE into the left hind paw (ipsilateral) at day 8 of oxaliplatin-treated mice induced a strong reduction of mechanical paw withdrawal latencies in wild type mice but not in TRPV1-deficient mice (Figure 2J).

### Example 7: 9-HODE activates the G2A-receptor (GPR132)

Sensitization of TRPV1 are commonly mediated via GPCR signaling pathways (Moriyama et al., 2005) (Sisignano et al., 2014) and was previously shown in a heterologous expression system that 9-HODE can activate the G2A receptor (GPR132) (Obinata et al., 2005). Accordingly, it was observed that HEK-cells overexpressing G2A showed increased intracellular calcium concentrations after stimulation with 9-HODE (Fig. 3A and 3B). The concentration-curve (10⁻⁸ to 10⁻⁵ M) for G2A transfected COS-1 cells and mock (empty vector) transfected COS-1 cells is shown in (Figure 3C).

To confirm the genetic deficiency of G2A in G2A-knockout mice, we determined G2A mRNA expression levels in lumbal DRGs in wild type and G2A knockout mice. As expected G2A-mRNA was detected in DRGs from wild type but not in DRGs from G2A-knockout mice (Fig. 3E). To determine whether or not the effects of 9-HODE towards TRPV1 sensitization are G2A-dependent, calcium imaging experiments using primary DRG cultures of wild type and G2A-deficient mice were performed. 9-HODE-induced TRPV1 sensitization was absent in DRG cultures of G2A-deficient mice (Fig. 3F and 3G).

### Example 8: 9-HODE induced TRPV1-sensitization is mediated by PKC but not PKA.

TRPV1 sensitization can be mediated by PKA or PKC (Schnizler et al., 2008; Bhave et al., 2003). To investigate the signaling pathway between G2A and TRPV1 in DRG neurons, we determined which protein kinases are involved in sensitization of TRPV1. Thus, we used inhibitors for PKA (H89; Figure 4A) and PKC (GF-109203X; Figure 4B) to test their effect on 9-HODE-induced TRPV1 sensitization. Incubation of DRG neurons with the PKA-inhibitor H89 inhibited the PKA-dependent TRPV1 sensitization caused by 8-bromo-cAMP but failed to supress the 9-HODE-induced TRPV1 sensitization (Figure 4A). However, the PKC-inhibitor GF109203X prevented TRPV1 sensitization caused by 9-HODE (Figure 4B). Bradykinin was used as positive control for PKC-dependent TRPV1 sensitization.

### Example 9: G2A-deficient mice have less pain in response to oxaliplatin treatment

The expression of G2A mRNA levels in DRGs was increased 10 days after oxaliplatin injection (Fig. 5A). A reduced mechanical hypersensitivity over the time period of 10 days after treatment with oxaliplatin could be observed in G2A-deficient mice compared to wild type mice (Figure 5B). Moreover, intraplantar injection of 9-HODE into the left hind paw at day eight after oxaliplatin treatment induced a strong reduction of mechanical pain thresholds in wildtype mice but not in G2A-deficient mice (Figure 5C).

### References

Basora et al (2003) 20-hydroxyeicosatetraenoic acid (20-HETE) activates mouse TRPC6 channels expressed in HEK293 cells. Journal of Biological Chemistry 278.34 : 31709-31716.
Bhave et al. (2003) Protein kinase C phosphorylation sensitizes but does not activate the capsaicin receptor transient receptor potential vanilloid 1 (TRPV1). Proceedings of the National Academy of Sciences of the United States of America 100:12480-12485.
Choudhary et al. (2003) Comparative expression profiling of 40 mouse cytochrome P450 genes in embryonic and adult tissues. Archives of Biochemistry and Biophysics 414:91-100.
Chavan et al., (2015) Functional Coupling of ATP-binding Cassette Transporter Abcb6 to Cytochrome P450 Expression and Activity in Liver. Journal of Biological Chemistry 290(12): 7871-7886.
Hershman et al. (2014) Prevention and management of chemotherapy-induced peripheral neuropathy in survivors of adult cancers: American Society of Clinical Oncology clinical practice guideline. Journal of clinical oncology: official journal of the American Society of Clinical Oncology 32:1941-1967.
Julius D (2013) TRP channels and pain. Annual review of cell and developmental biology 29:355-384.
Li et al. (2015) The Cancer Chemotherapeutic Paclitaxel Increases Human and Rodent Sensory Neuron Responses to TRPV1 by Activation of TLR4. The Journal of neuroscience : the official journal of the Society for Neuroscience 35:13487-13500.
Luo et al (1998). Cloning and expression of murine CYP2Cs and their ability to metabolize arachidonic acid. Archives of Biochemistry and Biophysics 357.1 : 45-57.
Moran et al. (2011) Transient receptor potential channels as therapeutic targets. Nature reviews. Drug discovery 10:601-620.
Moriyama et al. (2005) Sensitization of TRPV1 by EP1 and lP reveals peripheral nociceptive mechanism of prostaglandins. Molecular pain 1:3.
Murakami et al. (2004). G2A is a proton-sensing G-protein-coupled receptor antagonized by lysophosphatidylcholine. Journal of Biological Chemistry 279.41 : 42484-42491.
Nassini et al. P (2011) Oxaliplatin elicits mechanical and cold allodynia in rodents via TRPA1 receptor stimulation. Pain 152:1621-1631.
Obinata et al. (2005) Identification of 9- hydroxyoctadecadienoic acid and other oxidized free fatty acids as ligands of the G protein-coupled receptor G2A. The Journal of biological chemistry 280:40676-40683.
Pachman et al. (2011) Chemotherapy-induced peripheral neuropathy: prevention and treatment. Clinical pharmacology and therapeutics 90:377- 387.
Schnizler et al. (2008) Protein kinase A anchoring via AKAP150 is essential for TRPV1 modulation by forskolin and prostaglandin E2 in mouse sensory neurons. The Journal of neuroscience: the official journal of the Society for Neuroscience 28:4904-4917.
Sisignano et al. (2014) Mechanism-based treatment for chemotherapy-induced peripheral neuropathic pain. Nat Rev Neural.
Sisignano et al. (2014) TRP-channels as keyintegrators of lipid pathways in nociceptive neurons. Progress in lipid research 53:93-107.
Wang et al. (2008) Phospholipase C and protein kinase A mediate bradykinin sensitization of TRPA1: a molecular mechanism of inflammatory pain. Brain :a journal of neurology 131:1241-1251.
Wang et al. (2012) Discovering cylokines as targets for chemotherapy-induced painful peripheral neuropathy. Cytokine 59:3-9.
Wang et al. (2015) Modality-specific mechanisms of protein kinase C-induced hypersensitivity of TRPV1: S800 is a polymodal sensitization site. Pain 156:931-941.
Watanabe et al. (2003) Anandamide and arachidonic acid use epoxyeicosatrienoic acids to activate TRPV4 channels. Nature 424.6947 : 434-438.
Wen et al. (2012) 20-Hydroxyeicosatetraenoic acid (20-HETE) is a novel activator of transient receptor potential vanilloid 1 (TRPV1) channel. The Journal of biological chemistry 287:13868-13876.
Weng et al. (1998) DNA damage and stress inducible G protein-coupled receptor blocks cells in G2M. Proc Nail Acad Sci US A: 12334-9.

## Claims

1. An inhibitor of GPR132 (G protein coupled receptor 132) for use in preventing and/or treating chemotherapy-induced neuropathic pain in a subject, wherein said subject has received at least one platinum-containing chemotherapeutic agent.

2. The inhibitor of GPR132 for use of claim 1 wherein said at least one platinum-containing chemotherapeutic agent is oxaliplatin.

3. The inhibitor of GPR132 for use of claim 1 or 2 wherein said inhibitor reduces chemotherapy-dependent hyper-excitability of sensory neurons.

4. The inhibitor of GPR132 for use of any one of claims 1 to 3, wherein said inhibitor reduces chemotherapy-dependent sensitization of a transient receptor potential (TRP) channel.

5. The inhibitor of GPR132 for use of claim 4, wherein said transient receptor potential (TRP) channel is selected from the group consisting of: TRPV1, TRPA1, TRPM8, TRPV4, TRPC3 and TRPC6.

6. The inhibitor of GPR132 for use of claim 4 or 5, wherein said chemotherapy-dependent sensitization of a transient receptor potential (TRP) channel is mediated by oxidized lipids.

7. The inhibitor of GPR132 for use of claim 6, wherein said oxidized lipids are selected from the group consisting of: hydroxyoctadecadienoic acids (HODEs), dihydroxyoctadecenoic acids (DiHOMEs) and epoxyoctadecenoic acids (EpOMEs).

8. The inhibitor of GPR132 for use of claim 6 or 7, wherein said oxidized lipids are selected from the group consisting of: 9-HODE, 13-HODE, 9,10-DiHOME, 12,13-DiHOME, 9,10-EpOME and 12,13-EpOME.

9. The inhibitor of GPR132 for use of any one of claims 1 to 8 wherein said subject is a mammal, preferably a human.

10. The inhibitor of GPR132 for use of any one of claims 1 to 9 wherein the inhibitor is given to said subject by oral and/or parental administration.

11. The inhibitor of GPR132 for use of any one of claims 1 to 10 wherein said inhibitor is a peptide or protein, a ribozyme, an antibody, a small molecule, an inhibitory RNA molecule, an antisense oligonucleotide, a morpholino or a lipid, in particular lysophosphatidylcholine (LPC).

12. A pharmaceutical composition comprising an inhibitor of GPR132 wherein said inhibitor is a peptide or protein, a ribozyme, an antibody, a small molecule, an inhibitory RNA molecule, an antisense oligonucleotide, a morpholino or a lipid, in particular lysophosphatidylcholine (LPC).

13. The pharmaceutical composition of claim 12, further comprising a pharmaceutically acceptable carrier.

14. A method for identifying an inhibitor of GPR132 comprising the steps of
a) contacting a cell expressing GPR132 cultivated in the presence of an oxidized lipid with a compound suspected to be a GRP 132 inhibitor;
b) determining GPR132 activity after said contacting; and
c) comparing the determined GPR132 activity to GPR132 activity determined in a control cell, whereby an inhibitor of GPR132 is identified.

15. The method of claim 14, wherein said control cell is a cell expressing GPR132 cultivated in the presence of an oxidized lipid which has not been contacted to a compound suspected to be a GRP 132 inhibitor.
